(19) Europäisches Patentamt
European Patent Office
Office européen des brevets

(11) Veröffentlichungsnummer: **0 101 662**
**A2**

# (12) EUROPÄISCHE PATENTANMELDUNG

(21) Anmeldenummer: **83810273.9**

(22) Anmeldetag: **20.06.83**

(51) Int. Cl.³: **C 07 D 401/12**, A 01 N 43/50, A 01 N 43/40

(30) Priorität: **25.06.82 CH 3924/82**
**26.05.83 CH 2870/83**

(43) Veröffentlichungstag der Anmeldung: **29.02.84**
**Patentblatt 84/9**

(84) Benannte Vertragsstaaten: **AT BE CH DE FR GB IT LI NL**

(71) Anmelder: **CIBA-GEIGY AG, Patentabteilung Postfach, CH-4002 Basel (CH)**

(72) Erfinder: **Böger, Manfred, Wilhelm Glock-Strasse 14, D-7858 Weil am Rhein 5 (DE)**
Erfinder: **Drabek, Jozef, Dr., Benkenstrasse 12, CH-4104 Oberwil (CH)**

(54) **Benzoylparabansäuren.**

(57) Neue substituierte 1-Pyridyloxyphenyl-3-benzoyl-parabansäuren der Formel

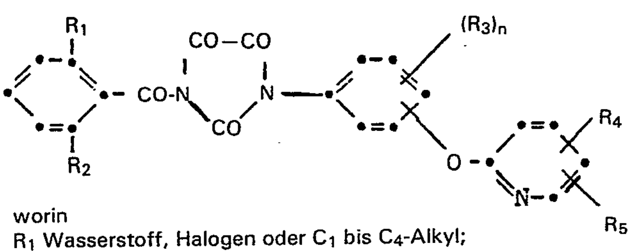

worin
$R_1$ Wasserstoff, Halogen oder $C_1$ bis $C_4$-Alkyl;
$R_2$ und $R_3$ unabhängig voneinander Halogen oder $C_1$ bis $C_4$-Alkyl;
$R_4$ Wasserstoff oder Halogen;
$R_5$ Halogen oder mit Fluor und/oder Chlor halogeniertes $C_1$ bis $C_4$-Alkyl: und
n eine Zahl 0, 1 oder 2
bedeuten, wobei die Pyridyloxygruppe an die 3- oder 4-Stellung der Phenylgruppe gebunden ist; Verfahren zur Herstellung dieser Verbindung sowie diese enthaltende Mittel zur Verwendung in der Schädlingsbekämpfung, vor allem zur Bekämpfung von Pflanzen und Tiere befallenden Insekten. Die neuen Verbindungen sind insbesondere wirksam gegen pflanzenschädigende Insekten, speziell als Ovizide.



ACTORUM AG

0101662



CIBA-GEIGY AG
Basel (Schweiz)

5-13984/1+2

Benzoylparabansäuren

Die vorliegende Erfindung betrifft neue substituierte 1-Pyridyloxy-phenyl-3-benzoyl-parabansäuren, Verfahren zu ihrer Herstellung und ihre Verwendung in der Schädlingsbekämpfung.

Die erfindungsgemässen 1-Pyridyloxyphenyl-3-benzoyl-parabansäuren haben die Formel I

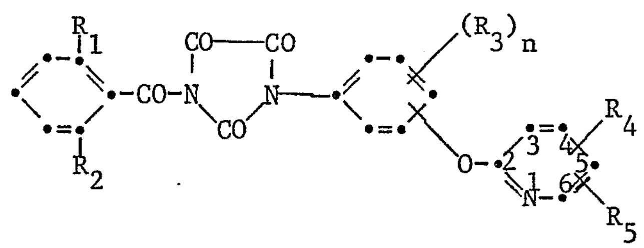

(I),

worin

$R_1$ Wasserstoff, Halogen oder $C_1$ bis $C_4$-Alkyl;
$R_2$ und $R_3$ unabhängig voneinander Halogen oder $C_1$ bis $C_4$-Alkyl;
$R_4$ Wasserstoff oder Halogen;
$R_5$ Halogen oder mit Fluor und/oder Chlor halogeniertes $C_1$ bis $C_4$-Alkyl; und
n eine Zahl 0, 1 oder 2

bedeuten, wobei die Pyridyloxygruppe an die 3- oder 4-Stellung der Phenylgruppe gebunden ist.

Bevorzugt sind erfindungsgemäss Verbindungen der Formel I, worin $R_1$ Wasserstoff, Fluor, Chlor, Brom oder Methyl und $R_2$ und $R_3$ unabhängig voneinander Fluor, Chlor, Brom oder Methyl, $R_4$ Wasserstoff oder Chlor und $R_5$ Chlor oder eine mit Fluor und/oder Chlor halogenierte Methyl- oder Aethylgruppe bedeuten. Wegen ihrer Wirkung als Schädlings-

BAD ORIGINAL

bekämpfungsmittel hervorzuheben sind insbesondere Verbindungen der Formel I, worin
$R_1$ Wasserstoff, Fluor oder Chlor; $R_2$ Fluor, Chlor oder Methyl; $R_3$ Fluor oder Chlor; $R_4$ Wasserstoff oder in 3- oder 6-Stellung am Pyridyloxyrest befindliches Chlor und $R_5$ eine mit Fluor und/oder Chlor perhalogenierte Methyl- oder Aethylgruppe bedeuten.

Von spezieller Bedeutung sind auch solche Verbindungen der Formel I, worin der Rest $R_5$, der sich bevorzugt in 5-Stellung am Pyridyloxyrest befindet, $-CF_3$, $-CF_2CCl_3$, $-CF_2CFCl_2$ oder $-CF_2CF_2Cl$ bedeutet. Hervorzuheben sind ferner diejenigen Verbindungen der Formel I, worin $R_1$ und $R_2$ unabhängig voneinander Fluor oder Chlor, vorzugsweise beide Reste $R_1$ und $R_2$ Fluor, bedeuten. Von besonderem Interesse sind daneben Verbindungen der Formel I, bei denen $R_3$ Chlor bedeutet.

Die Verbindungen der Formel I können analog an sich bekannten Verfahren (vgl. z.B. J.Agr.Food Chem. 21, No. 3, 348 - 349 (1973) und Britische Patentschrift Nr. 1 324 293) hergestellt werden.

So kann man z.B. eine Verbindung der Formel I erhalten, indem man einen Pyridyloxyphenylbenzoylharnstoff der Formel II

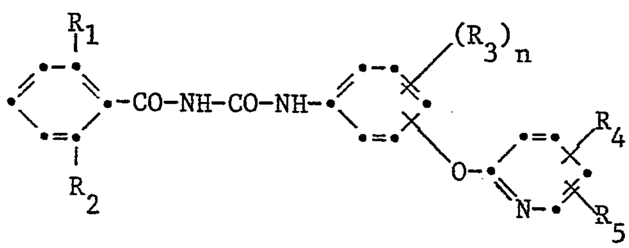

(II)

mit Oxalylchlorid umsetzt. In Formel II haben die Reste $R_1$ bis $R_5$ und n die unter Formel I vorstehend angegebenen Bedeutungen.

Das erfindungsgemässe Verfahren kann vorzugsweise unter normalem Druck und in Gegenwart eines organischen Lösungs- oder Verdünnungsmittels durchgeführt werden. Als Lösungs- oder Verdünnungsmittel

eignen sich z.B. Ather und ätherartige Verbindungen, wie Dipropyläther, Dibutyläther, Dioxan, Dimethoxyäthan und Tetrahydrofuran; N,N-dialkylierte Carbonsäureamide; aliphatische, aromatische sowie vorzugsweise halogenierte Kohlenwasserstoffe, insbesondere Benzol, Toluol, Xylol, Chloroform, Methylenchlorid, 1,2-Dichloräthan, Tetrachlorkohlenstoff und Chlorbenzol; Dimethylsulfoxid sowie Ketone, z.B. Aceton, Methyläthylketon und Methylisobutylketon. Das Verfahren wird im allgemeinen bei einer Temperatur von -10 bis 200°C, vorzugsweise zwischen 10 und 150°C, durchgeführt, insbesondere beim Siedepunkt des verwendeten inerten Lösungsmittels.

Die Ausgangsstoffe der Formel II sind bekannt bzw. können analog bekannten Verfahren, wie z.B. in der deutschen Offenlegungsschrift 27 48 636 aufgeführt, hergestellt werden.

Aus der britischen Patentschrift Nr. 1 324 293 sind 1-Phenyl-3-benzoylparabansäuren mit pestizider Wirkung bekannt. Bei den erfindungsgemässen Verbindungen handelt es sich demgegenüber um neuartige substituierte 1-(2-Pyridyloxyphenyl)-3-benzoylparabansäuren, die überraschenderweise erhöhte Wirksamkeit als Schädlingsbekämpfungsmittel, insbesondere als Insektizide im Pflanzenschutz, aufweisen. Ein besonderer Vorteil der erfindungsgemässen Verbindungen der Formel I ergibt sich aus ihrer sehr geringen Warmblütertoxizität bei guter Pflanzenverträglichkeit, verbunden mit vorteilhaften Löslichkeitseigenschaften.

Insbesondere eignen sich die Verbindungen der Formel I zur Bekämpfung von Insekten der Ordnungen: Lepidoptera, Coleoptera, Homoptera, Heteroptera Diptera, Thysanoptera, Orthoptera, Anoplura, Siphonaptera, Mallophaga, Thysanura, Isoptera, Psocoptera und Hymenoptera, sowie von Vertretern der Ordnung Akarina, insbesondere Milben und Zecken.

Neben ihrer Wirkung gegenüber Fliegen, wie z.B. Musca domestica, und Mückenlarven können Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte ovizide und larvizide Wirkung gegen Insekten, insbesondere gegen Eier und Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/ oder Akariziden wesentlich verbreitern und an gegebene Umstände anpassen.

Als Zusätze kommen z.B. folgende Wirkstoffe in Betracht: organische Phosphorverbindungen, Nitrophenole und Derivate, Formamidine, Harnstoffe, Carbamate, chlorierte Kohlenwasserstoffe, Pyrethroide und Bacillus thuringiensis-Präparate.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

BAD ORIGINAL

Die gute insektizide Wirkung der erfindungsgemässen Verbindungen der Formel I entspricht einer Abtötungsrate (Mortalität) von mindestens 50-60% der erwähnten Schadinsekten.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I, bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

BAD ORIGINAL

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände verwendet, werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind als Tenside auch die Fettsäuremethyl-taurinsalze sowie modifizierte und nicht-modifizierte Phospholipide zu erwähnen.

Häufiger werden jedoch sogenannte synthetische Tenside verwendet,

BAD ORIGINAL

insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit etwa 8 - 22 C-Atomen. Alkylarylsulfonate sind z.B. die Na-, Ca- oder Triäthanolaminsäure der Dodecylbenzolsulfonsäure, der Dibutylnaphthalinsulfonsäure oder eines Naphthalinsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte,

BAD ORIGINAL

Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1981. H. Stache, "Tensid-Taschenbuch", Carl Hauser Verlag, München/Wien, 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere, 0,1 bis 20 %, eines Tensides. Während als Handelsware eher konzentrierte Mittel, bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

BAD ORIGINAL

Formulierungsbeispiele für feste Wirkstoffe der Formel I resp. Kombinationen dieser Wirkstoffe mit andern Insektiziden oder Akariziden (% = Gewichtsprozent)

| Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination werden mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Aus diesem Konzentrat können durch Verdünnen mit Wasser Emulsionen jeder gewünschten Konzentration hergestellt werden.

| Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

BAD ORIGINAL

Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie-Umhüllungs-Granulate.

Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

BAD ORIGINAL

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung von 1-[4-(5-Trifluormethyl-2-pyridyloxy)-phenyl]-3-(2,6-difluorbenzoyl)-parabansäure:

12,6 g N-4-(5-Trifluormethyl-2-pyridyloxy)-phenyl-N-2,6-difluorbenzoylharnstoff und 4,4 g Oxalylchlorid werden in 100 ml 1,2-Dichloräthan gelöst und 20 Stunden am Rückfluss erhitzt. Dann wird das Lösungsmittel abdestilliert und der Rückstand in einem Gemisch von Toluol/Hexan (Volumenteile 1:1) angeschlämmt und abgenutscht. Nach Umkristallisation aus Toluol (80°C) erhält man die Titelverbindung der Formel

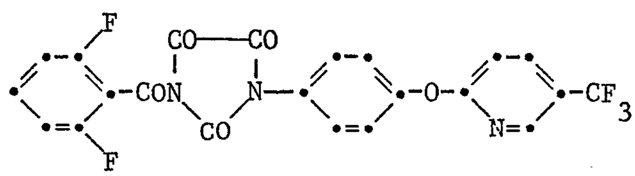

als weisses kristallines Pulver mit einem Schmelzpunkt von 152-154°C (Verbindung Nr. 1).

Analog der vorstehend beschriebenen Arbeitsweise werden die folgenden Verbindungen der Formel I herstellt:

Verbindung Nr. 2

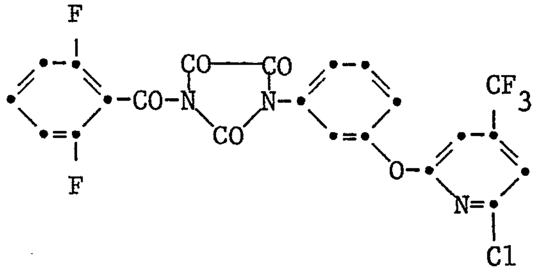

Smp. 73-75°C

BAD ORIGINAL

0101662



Verbindung Nr. 3

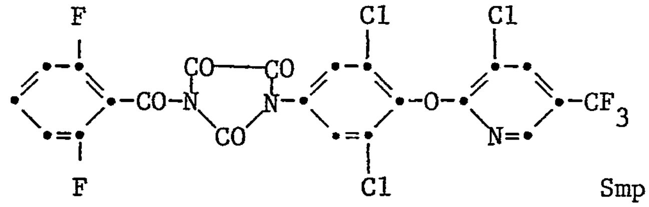

Smp.157-158°C

Verbindung Nr. 4

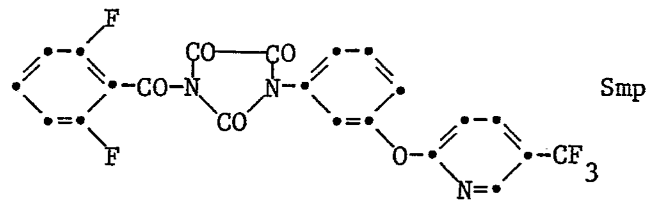

Smp. 77-80°C

Verbindung Nr. 5

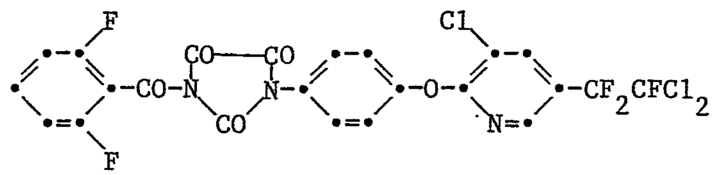

Smp.166-168°C

Verbindung Nr. 6

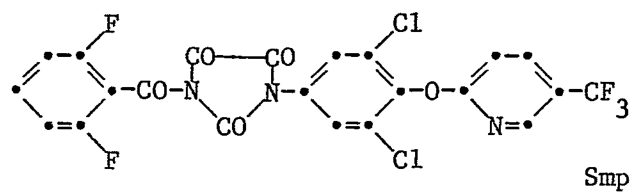

Smp. 143-146°C

In entsprechender Weise sind die folgenden Verbindungen der Formel I erhältlich:

Verbindung Nr. 7

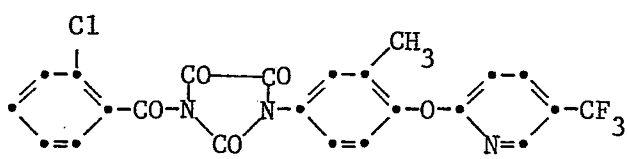

Verbindung Nr. 8 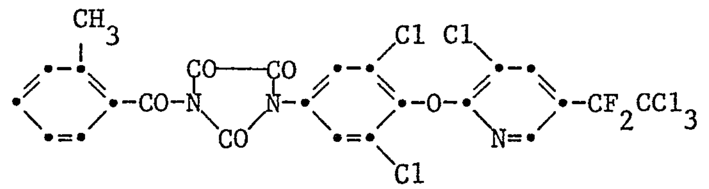 ,

Verbindung Nr. 9 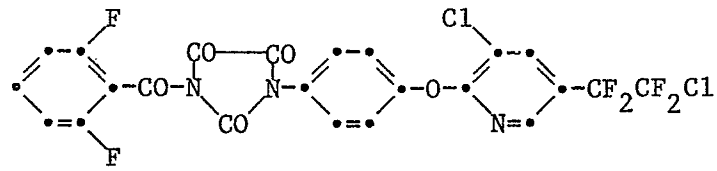

Verbindung Nr. 10 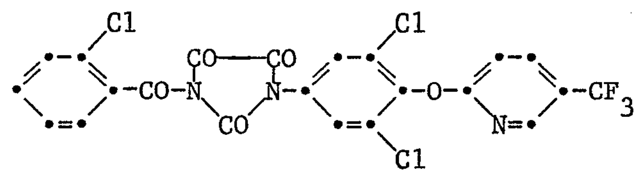

<u>Beispiel 2:</u> <u>Wirkung gegen Musca domestica:</u> Je 50 g frisch zubereitetes Nährsubstrat für Maden wird in Becher eingewogen. Von einer 1 Gew.%igen acetonischen Lösung des betreffenden Wirkstoffes wird eine bestimmte Menge auf das in den Bechern befindliche Nährsubstrat pipettiert. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss dem vorstehenden Beispiel 1 zeigen gute Wirkung im obigen Test.

Beispiel 3: Wirkung gegen Lucilia sericata: Zu 9 ml eines Zuchtmediums werden bei 50°C 1 ml einer 0,5 % Aktivsubstanz enthaltenden wässrigen Lösung gegeben. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Verbindungen der Formel I gemäss dem vorstehenden Beispiel 1 zeigen in diesem Test gute Wirkung gegen Lucilia sericata.

Beispiel 4: Wirkung gegen Aëdes aegypti: Auf die Oberfläche von 150 ml Wasser, das sich in einem Behälter befindet, wird so viel einer 0,1 %igen acetonischen Lösung des Wirkstoffes pipettiert, dass Konzentrationen von je 800 und 400 ppm erhalten werden. Nach Verdunsten des Acetons wird der Behälter mit 30 bis 40 2-tägigen Aëdes-Larven beschickt. Nach 1, 2 und 5 Tagen wird die Mortalität geprüft. Verbindungen der Formel I gemäss dem vorstehenden Beispiel 1 zeigen gute Wirkung im obigen Test.

Beispiel 5: Insektizide Frassgift-Wirkung: Ca. 25 cm hohe eingetopfte Baumwollpflanzen werden mit wässrigen Wirkstoffemulsionen besprüht, die den Wirkstoff in Konzentrationen von 400, 200, 100, 50, 12,5 und 0,75 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und 60 % relativer Luftfeuchtigkeit durchgeführt. Nach 120 Stunden wird die %-Mortalität der Test-Insekten bestimmt.

Eine 80 - 100%ige Wirkung gegen Spodoptera littoralis-Larven zeigt Verbindung Nr. 2 bei 12,5 ppm, Verbindung Nr. 3 bei 0,75 ppm, Verbindung Nr. 4 bei 200 ppm und Verbindung Nr. 5 bei 100 ppm.

Eine 80 - 100 %ige Wirkung gegen Heliothis virescens-Larven zeigen Verbindung Nr. 2 bei 50 ppm, Verbindung Nr. 3 bei 0,75 ppm und die Verbindungen Nr. 4 sowie Nr. 5 bei 100 ppm.

Beispiel 6: Wirkung gegen Epilachna varivestis: Etwa 15-20 cm hohe Phaseolus vulgaris-Pflanzen (Buschbohnen) werden mit wässrigen, den zu prüfenden Wirkstoff in Konzentrationen von 400 ppm bzw. 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28°C und 60% relativer Luftfeuchtigkeit durchgeführt.

Nach 2 und 3 Tagen wird die akute Wirkung (% Mortaliät) bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Verbindungen der Formel I gemäss dem vorstehenden Beispiel 1 zeigen gute Wirkung im obigen Test.

Beispiel 7: Ovizide Wirkung auf Heliothis virescens:
Entsprechende Mengenanteile einer benetzbaren pulverförmigen Formulierung, enthaltend 25 Gew.% des zu prüfenden Wirkstoffes, werden mit jeweils soviel Wasser vermischt, dass sich wässrige Emulsionen von ansteigender Wirkstoffkonzentration ergeben.

In diese wirkstoffhaltigen Emulsionen werden eintägige Eigelege von Heliothis auf Cellophan während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird

die Schlupfrate im Vergleich zu unbehandelten Kontrollen festgestellt. Zur Auswertung wird die zur 100 %igen Abtötung der Eier erforderliche minimale Wirkstoffkonzentration bestimmt.

Verbindungen der Formel I gemäss dem vorstehenden Beispiel 1 zeigen gute Wirkung in obigem Test.

Beispiel 8: Wirkung auf Laspeyresia pomonella (Eier):

Abgelegte Eier von Laspeyresia pomonella, die nicht älter als 24 Stunden sind, werden auf Filterpapier für 1 Minute in eine acetonischwässrige Lösung, enthaltend 12,5, 50, 100 und 400 ppm des zu prüfenden Wirkstoffes, eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Verbindung Nr. 1 gemäss dem vorstehenden Beispiel 1 zeigt 80 - 100 %ige Wirkung in obigem Test bei 12,5 ppm; Verbindung Nr. 5 zeigt 80 - 100 %ige Wirkung bei 400 ppm.

Beispiel 9: Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 1,0 Gew.% des zu prüfenden Wirkstoffes, eingetaucht.

Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten

Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss dem vorstehenden Beispiel 1 zeigen eine gute reproduktionsreduzierende Wirkung im obigen Test.

Beispiel 10: Wirkung gegen Anthonomus grandis:

Zwei eingetopfte Baumwollpflanzen im 6-Blattstadium werden mit einer wässrigen, benetzungsfähigen Emulsions-Zubereitung enthaltend 12,5, 50, 100 und 400 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung gegenüber unbehandelten Kontrollansätzen erfolgt nach 2, 3, 4 und 5 Tagen unter Zugrundelegung der prozentualen Mortalität der eingesetzten Testtiere (% Rückenlage) sowie der Antifeedant-Wirkung.

Die Verbindung Nr. 3 gemäss Beispiel 1 zeigte bei 12,5 ppm eine Wirkung von 80 - 100 % in diesem Test.

Patentansprüche:

1. Verbindung der Formel I

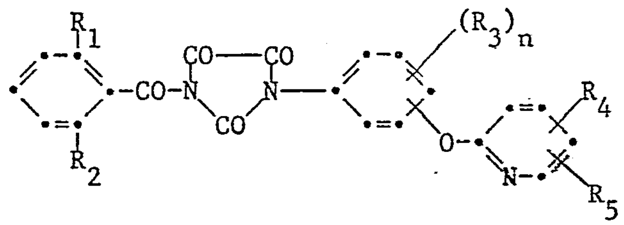

(I),

worin

$R_1$ Wasserstoff, Halogen oder $C_1$ bis $C_4$-Alkyl;

$R_2$ und $R_3$ unabhängig voneinander Halogen oder $C_1$ bis $C_4$-Alkyl;

$R_4$ Wasserstoff oder Halogen;

$R_5$ Halogen oder mit Fluor und/oder Chlor halogeniertes $C_1$ bis $C_4$-Alkyl; und

n eine Zahl 0, 1 oder 2

bedeuten, wobei die Pyridyloxygruppe an die 3- oder 4-Stellung der Phenylgruppe gebunden ist.

2. Verbindungen der Formel I gemäss Anspruch 1, dadurch gekennzeichnet, dass

$R_1$ Wasserstoff, Fluor, Chlor, Brom oder Methyl;

$R_2$ und $R_3$ unabhängig voneinander Fluor, Chlor, Brom oder Methyl;

$R_4$ Wasserstoff oder Chlor; und

$R_5$ Chlor oder eine mit Fluor und/oder Chlor halogenierte Methyl- oder Aethylgruppe bedeuten.

3. Verbindungen der Formel I gemäss Anspruch 2, dadurch gekennzeichnet, dass

$R_1$ Wasserstoff, Fluor oder Chlor;

$R_2$ Fluor, Chlor oder Methyl;

$R_3$ Fluor oder Chlor;

$R_4$ Wasserstoff, 3-Chlor oder 6-Chlor; und

$R_5$ eine mit Fluor und/oder Chlor perhalogenierte Methyl- oder Aethylgruppe bedeuten.

4. Verbindung der Formel I gemäss Anspruch 3, dadurch gekennzeichnet, dass $R_5$ $-CF_3$, $-CF_2CCl_3$, $-CF_2CFCl_2$ oder $-CF_2CF_2Cl$ bedeutet.

5. Verbindung der Formel I gemäss Anspruch 1 bis 4, dadurch gekennzeichnet, dass sich der Rest $R_5$ in 5-Stellung befindet.

6. Verbindung der Formel I gemäss Anspruch 1 bis 5, dadurch gekennzeichnet, dass $R_1$ und $R_2$ unabhängig voneinander Fluor oder Chlor bedeuten.

7. Verbindung der Formel I gemäss Anspruch 6, dadurch gekennzeichnet, dass $R_1$ und $R_2$ Fluor bedeuten.

8. Verbindung der Formel I gemäss Anspruch 1 bis 7, dadurch gekennzeichnet, dass $R_3$ Chlor bedeuten.

9. Verbindung gemäss Anspruch 7 der Formel

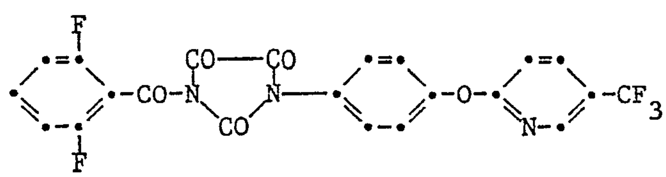

10. Verbindung gemäss Anspruch 7 der Formel

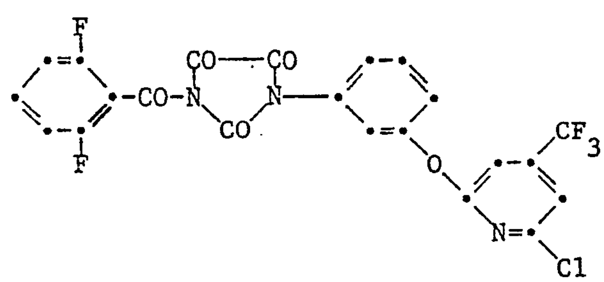

11. Verbindung gemäss Anspruch 7 der Formel

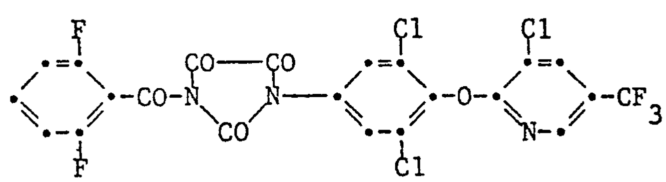

12. Verbindung gemäss Anspruch 7 der Formel

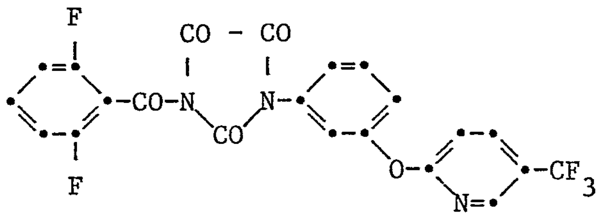

13. Verfahren zur Herstellung einer Verbindung der Formel I gemäss Anspruch 1 bis 12, dadurch gekennzeichnet, dass man eine Verbindung der Formel II

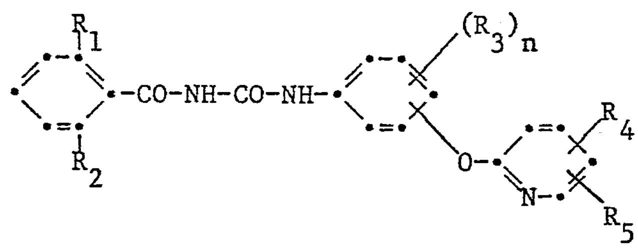

(II),

worin $R_1$ bis $R_5$ und n die in den Ansprüchen 1 bis 8 angegebenen Bedeutungen haben, mit Oxalylchlorid umsetzt.

14. Schädlingsbekämpfungsmittel, welches als aktive Komponente eine Verbindung gemäss den Ansprüchen 1 bis 12 zusammen mit geeigneten Trägern und/oder anderen Zuschlagstoffen enthält.

15. Verwendung einer Verbindung gemäss den Ansprüchen 1 bis 12 zur Bekämpfung von Schädlingen, vorzugsweise von Insekten.

16. Verwendung gemäss Anspruch 15 zur Bekämpfung von fressenden, pflanzenschädigenden Insekten.

17. Verwendung gemäss Anspruch 16 als Ovizid.

FO 7.5 EIC/as*/rn* /rz*